# EUROPÄISCHE PATENTSCHRIFT

(11) **EP 1 379 569 B1**
(45) Veröffentlichungstag und Bekanntmachung des Hinweises auf die Patenterteilung: **20.09.2006**
(21) Anmeldenummer: 02726142.9
(22) Anmeldetag: 06.03.2002
(51) Int. Cl.: C08G 18/76, C07C 263/20

(54) **VERFAHREN ZUR HERSTELLUNG VON MDI, INSBESONDERE VON 2,4'-MDI**
METHOD FOR PRODUCING MDI, ESPECIALLY 2,4'-MDI
PROCEDE DE PRODUCTION DE MDI, EN PARTICULIER DE 2,4'-MDI

(30) Priorität: 08.03.2001 DE 10111337
(43) Veröffentlichungstag der Anmeldung: 14.01.2004
(73) Patentinhaber: BASF Aktiengesellschaft, 67056 Ludwigshafen (DE)
(72) Erfinder: REIF, Martin, 67354 Römerberg (DE); BRUCHMANN, Bernd, 67251 Freinsheim (DE); POHL, Siegmund, 49356 Diepholz (DE); BETTENDORF, Carl, B-2950 Kapellen (DE); PLAUMANN, Heinz, Flat Rock, MI 48134 (US); THIELE, Kai, 01987 Schwarzheide (DE)
(86) Internationale Anmeldenummer: PCT/EP2002/002426
(87) Internationale Veröffentlichungsnummer: WO 2002/070581

(56) Entgegenhaltungen:
- EP-A- 0 003 303
- EP-A- 0 109 931
- FR-A- 2 024 209
- US-A- 4 259 526

## Beschreibung

Die Erfindung betrifft ein Verfahren zur Herstellung von MDI, umfassend die Schritte A) Umsetzung von Anilin und Formaldehyd in Gegenwart einer Säure als Katalysator zu Methylen(diphenyl-diaminen) und Polymethylen-polyphenylen-polyaminen, wobei das molare Verhältnis von Säure zu Amin 0,2 oder kleiner ist, B) Umsetzung des in Schritt A) erhaltenen Gemisches mit Phosgen zu Methylen(diphenyldiisocyanaten) und Polymethylen-polyphenylenpolyisocyanaten, C) Auftrennen des Gemisches aus Schritt B) in Monomer-MDI und PMDI und D) Auftrennen des in Schritt C) erhaltenen Monomer-MDI zur Gewinnung von 2,4'-MDI.

Aromatische Isocyanate sind wichtige und vielseitige Rohstoffe für die Polyurethanchemie. Hierbei sind Toluylendiisocyanat (TDI) und MDI die wichtigsten technischen Isocyanate.

Der allgemeine Begriff "MDI" wird im Fachgebiet und im Rahmen dieser Anmeldung als Oberbegriff für Methylen(diphenyldiisocyanate) und Polymethylen-polyphenylen-polyisocyanate verwendet. Der Begriff Methylen(diphenyldiisocyanat) umfasst die Isomere 2,2'- Methylen(diphenyldiisocyanat), 2,4'- Methylen(diphenyldiisocyanat) (2,4'-MDI) und 4,4'- Methylen(diphenyldiisocyanat) (4,4'-MDI). Zusammenfassend werden diese Isomere als "Monomer-MDI bezeichnet". Der Begriff Polymethylen-polyphenylen-polyisocyanate umfasst das sogenannte "Polymer-MDI" (PMDI), das Monomer-MDI und höhere Homologe des Monomer-MDI enthält.

Kommerziell sind von MDI verschiedene Isomere bzw. Isomerenmischungen erhältlich, neben 4,4'-MDI werden 4,4'-/2,4'-MDI-Mischungen und PMDI angeboten. Mit diesen verschiedenen Isocyanaten ist zwar eine große Vielfalt an PU-Polymeren mit unterschiedlichen Strukturen und Eigenschaften herstellbar, es gibt aber dennoch einige Einschränkungen: Polymer-MDI ist ein komplexes Gemisch unterschiedlicher MDI-Oligomerer (im allgemeinen N-Kern MDI, mit N = 2 - >10 und MDI-Isomerer (so gibt es z.B. 7 verschiedene 3-Kern MDI Isomere). Diese Produkte sind technisch von hohem Nutzen, ein gezielter Aufbau einzelner, definierter chemischer Strukturen ist aber nicht möglich. Ein definierter Aufbau von Polyurethanstrukturen ist aber essentielle Voraussetzung für das Erreichen bestimmter, gewünschter anwendungstechnischer Eigenschaften.

Monomer-MDI Typen (4,4'-/2,4'-MDI) sind, aufgrund ihrer Difunktionalität, für einen definierten Strukturaufbau besser als PMDI geeignet, wobei 4,4'-MDI zwei gleich reaktive Isocyanat-gruppen enthält. Aus diesem Grund ist jedoch bei 4,4'-MDI eine gezielte Funktionalisierung an nur einer Isocyanatfunktion praktisch nicht möglich, da eine Zweitaddition nur unwesentlich ungünstiger ist, man erhält folglich bei Additionsreaktionen üblicherweise Gemische.

Es ist daher wünschenswert ein 2-Kern MDI mit zwei unterschiedlich reaktiven Isocyanat-gruppen bereit zu stellen, um zu neuen Eigenschaftsprofilen zu kommen. 2,4'-MDI ist ein Molekül, das diese Anforderungen erfüllt. Hier ist eine Reaktion in 4-Position aus sterischen Gründen gegenüber einer Reaktion in 2-Position deutlich bevorzugt, ein gezielter Strukturaufbau damit möglich.

Im Stand der Technik sind bereits Vorteile von reinem 2,4'-MDI bzw. 2,4'-MDI reichen MDI-Gemischen diskutiert.
- A.M. Sarpeshkar, P.H. Markusch, R.L. Cline "Designing Polyurethane Elastomers with low Compression Sets" in: Polyurethanes Conference 2000, Proceedings, Tagungsband, Tagung vom 8 bis 11. Oktober 2000 in Boston, Massachusetts. Hier wird für die Herstellung von PU-Elastomeren mit besonderen Eigenschaften der positive Einfluss von 2,4'-MDI auf den Druckverformungsrest beschrieben.
- EP-B-0676434 beschreibt, dass in PU-Weich(form)schaumsystemen z.B. durch den Einsatz von 2,4'-MDI das sonst verwendete 2,4-TDI/2,6-TDI Gemisch ersetzt werden konnte.
- EP-B-0431331 offenbart den Einsatz von 2,4'-MDI in hitzehärtbaren 1-K PU-Systemen.
- EP-A-0572994 beschreibt die Herstellung von Uretdiongruppen aufweisenden Polyisocyanaten.
- DE-A-19904444 und WO 97/02304 beschreiben die Synthese von Dendrimeren und hochverzweigten Polyurethanen.

Aus vorstehenden Erläuterungen wird deutlich, dass insbesondere von PU-Systemherstellern ein Bedarf an 2,4'-MDI besteht. Trotzdem ist 2,4'-MDI - obwohl dieses Molekül seit langem bekannt ist - nicht in isomerenreiner Form kommerziell in technischem Maßstab erhältlich. Lediglich 2,4'-MDI/4,4'-MDI Gemische (z.B. 50/50 Lupranat® MI der BASF, sowie 2,4'-MDI reiche Polymer-MDI Prepolymere, z.B. Rubinat® 9483 von Huntsman) sind kommerziell erhältlich.

In allen großtechnisch relevanten Herstellverfahrert wird MDI durch Phosgenierung von Methylen(diphenyldiamin) (MDA) produziert. Die Synthese geschieht in einem zweistufigen Prozess. Zunächst wird Anilin mit Formaldehyd zu einem Gemisch aus oligomeren und isomeren Methylen(diphenyldiaminen) und Polymethylen-polyphenylen-polyaminen, dem sogenannten Roh-MDA kondensiert. Dieses Roh-MDA wird anschließend in einem zweiten Schritt mit Phosgen in an sich bekannter Weise zu einem Gemisch der entsprechenden oligomeren und isomeren Methylen(diphenyldiisocyanate) und Polymethylen-polyphenylen-polyisocyanate, dem sogenannten Roh-MDI umgesetzt. Hierbei bleibt die Isomeren- und Oligomerenzusammensetzung unverändert. Meist wird dann ein Teil der 2-Kern Verbindungen in einem weiteren Verfahrensschritt (z.B. durch Destillation oder Kristallisation) abgetrennt, wobei Polymer-MDI (PMDI) als Rückstand verbleibt.

Aus vorstehenden Erläuterungen wird klar, dass bei den Produktionsanlagen des Stands der Technik - bei sonst gleichbleibendem Produktmix, d.h. insbesondere bei sonst gleichbleibenden Mengen an produzierten 2,4'-/4,4'-MDI Mischungen - die zusätzliche Produktion von reinem 2,4'-MDI eine Reduzierung des 2,4'-MDI Gehaltes im PMDI zur Folge hat, da größere Mengen von 2,4'-MDI aus dem Roh-MDI abgetrennt werden. Aus anwendungstechnischen Gründen ist aber eine veränderte Zusammensetzung des PMDI nicht erwünscht, da sie die anwendungstechnischen Eigenschaften der daraus hergestellten PU-Systeme nachteilig beeinflussen kann. Für die technische PMDI-Herstellung ist es also wesentlich, dass die Zusammensetzung des PMDI konstant bleibt, somit erklärt sich, warum 2,4'-MDI bisher nicht kommerziell erhältlich ist.

Aufgabe der vorliegenden Erfindung war es deshalb ein Verfahren zur MDI Herstellung bereitzustellen, das es erlaubt, 2,4'-MDI zu erhalten, ohne dass die Produktionskosten des MDI-Verfahrens, insbesondere für den Schritt der Phosgenierung, wesentlich erhöht werden und ohne dass die Zusammensetzung des Produktmixes der Anlage verändert wird. Unter Produktmix der Anlage wird insbesondere die Zusammensetzung des ausgetragenen PMDI verstanden und die Zusammensetzung und Menge des ausgetragenen Monomer-MDI-Gemisches.

Die Anforderung, 2,4'-MDI herzustellen, ohne die Produktionskosten des Verfahrens zu erhöhen ist besonders vor dem Hintergrund zu sehen, dass es sich bei den großtechnischen MDI-Anlagen um komplizierte, kontinuierlich arbeitende Anlagen handelt, an die sehr hohe Anforderungen an Verfügbarkeit und Zuverlässigkeit gestellt werden. Eine weitere Aufgabe der Erfindung war es daher, ein Verfahren bereit zu stellen, das mit geringem technischem Aufwand auch in vorhandenen technischen MDI-Produktionsanlagen durchzuführen ist, ohne dass die Verfügbarkeit und Zuverlässigkeit des Verfahrens gefährdet wird.

In der Literatur sind zwar durchaus Vorteile und Verfahren zur Herstellung von 2,4'-MDI beschrieben, ein einfaches, kostengünstiges Verfahren zur Herstellung von 2,4'-MDI, das die Zusammensetzung des Koppelproduktes PMDI nicht verändert, ist bisher aber nicht bekannt.

DE-A-2930411 beschreibt ein Gemisch aus 2-Kern-MDI Isomeren mit einem hohen Gehalt an 2,2'-MDI und 2,4'-MDI als Lösungsmittel für Farbstabilisatoren, Antioxidantien, Flame-Retardants usw..

BE 735258 offenbart ein MDA-Verfahren unter Einsatz von sehr hohen Säuremengen wobei MDA mit einem möglichst geringen 2,4'-MDA Anteil erzeugt werden soll. Die eingesetzten Säuremengen sind für ein wirtschaftlich durchführbares Verfahren zu hoch, außerdem erhält man wenig des gewünschten 2,4'-Isomeren.

DE-A-26 31 168 beschreibt ein Verfahren zur Herstellung von Diisocyanatodiphenylmethan-Isomeren mit einem eingestellten Gehalt an Chlorverbindungen. Es erfolgt die Herstellung von sehr reinem 2-Kern MDI durch eine komplizierte Destillationsfolge. Im Rahmen der Versuche wird auch die Herstellung von reinem 2,4'-MDI. beschrieben. Ziel der Versuche war aber die Herstellung von 2-Kern MDI mit einem geringen Chlorgehalt. Eine Lehre zur Herstellung von größeren Mengen 2,4'-MDI ohne Änderung der Zusammensetzung des entsprechenden PMDI offenbart DE-A-26 31 168 aber nicht.

US-A-3892634, EP-A-0482490, JP-A-2951782, US-A-772790 und DE-A-2532722 beschreiben Destillation und Aufreinigung von 2-Kern MDI mit dem Ziel, möglichst helles bzw. möglichst chlorarmes Monomer-MDI zu erhalten. Bevorzugt ist die Gewinnung von reinem 4,4'-MDI. Bei diesen Stofftrennungen bzw. Destillationen treten zwar auch Stoffströme auf, die im 2,4'-MDI Gehalt angereichert sind, die Herstellung von reinem 2,4'-MDI war jedoch nicht Gegenstand dieser Erfindungen.

US-A-3,362,979 beschreibt die Herstellung von MDI-Mischungen mit hohem 2,4'-MDI Gehalt. Für die Synthese der MDA-Vorstufe werden anstelle der HCl aber spezielle Feststoffkatalysatoren eingesetzt. Die Herstellung von reinem 2,4'-MDI wird nicht beschrieben.

RU 2058303 beschreibt die Herstellung eines MDI-Gemisches mit einem höheren 2,4'-MDI Anteil. Hierfür wird aber die Anilinkondensation auf Basis der Dimethyl- bzw. Diethylacetale des Formaldehyds anstelle von reinem Formaldehyd in Gegenwart von HCl durchgeführt. Das Verfahren erfordert zusätzliche Einsatzstoffe, was der Forderung nach einem möglichst einfachen und kostengünstigen Verfahren widerspricht.

JP 06009539 beschreibt MDI-Gemische mit einem hohen Gehalt an 2,4'-MDI. Als Vorteil wird beschrieben, dass diese auch noch bei -10°C flüssig sind und nicht kristallisieren.

Weiterhin gibt es viele Druckschriften, die Abtrennung und Reinigung von 4,4'-MDI beschreiben..Prozessbedingt fallen dabei immer Prozessströme an, die einen höheren Gehalt an 2,4'-MDI aufweisen. Es ist aber nicht Gegenstand dieser Erfindungen, 2,4'-MDI zur Verfügung zu stellen. Beispiele dafür sind unter anderem BE 884805, US-A-470,400, DE-A-21051193, DE-A-2425658, WO 98/25889 und DE-A-2532722.

Die Aufgabe der vorliegenden Erfindung konnte überraschenderweise dadurch gelöst werden, indem man auf der Stufe der MDA-Synthese die Menge der als Katalysator verwendeten Säure erniedrigt und das erhaltene Roh-MDA dann zu Roh-MDI phosgeniert und anschließend einer speziellen Trennfolge, bevorzugt Destillationsfolge, zur Abtrennung von 2,4'-MDI unterwirft.

Gegenstand der Erfindung ist daher ein Verfahren zur Herstellung von MDI gemäß Anspruch 1 und den abhängigen Ansprüchen 2-8.

Zur im Schritt A) beschriebenen Umsetzung von Anilin mit Formaldehyd zu Methylen(diphenyldiaminen) und Polymethylen-polyphenylen-polyaminen, wobei dieses Gemisch als "Roh-MDA" bezeichnet wird, werden die Edukte üblicherweise in einer geeigneten Mischvorrichtung, wie beispielsweise in Mischpumpen, Düsen oder statischen Mischern, vermischt und in einer geeigneten Reaktionsvorrichtung, wie beispielsweise in Rohrreaktoren, Rührreaktoren und Reaktionskolonnen oder deren Kombinationen, umgesetzt. Die Umsetzungstemperatur beträgt im allgemeinen zwischen 20 und 200°C, bevorzugt zwischen 30 und 140°C.

Der Umsetzung von Schritt A) erfolgt in Gegenwart einer Säure als Katalysator, wobei der Katalysator bevorzugt im Gemisch mit Anilin zugegeben wird. Bevorzugte Katalysatoren sind Mineralsäuren, wie beispielsweise Salzsäure, Schwefelsäure und Phosphorsäure. Ebenfalls können Gemische von Säuren verwendet werden. Salzsäure ist besonders bevorzugt. Wird als Katalysator Chlorwasserstoff verwendet, so kann dieser auch gasförmig eingesetzt werden. Im Verfahren der vorliegenden Erfindung wird die Katalysatormenge so gewählt, dass sich ein molares Verhältnis von Säure/Anilin (S/A) ≤ 0,2, bevorzugt < 0,16 und besonders bevorzugt kleiner 0,1 ergibt.

In einer bevorzugten Ausführungsform wird die Umsetzung von Schritt A) in wässrigem Medium mit HCl als Katalysator durchgeführt. Ferner kann die Umsetzung in Gegenwart eines Lösungsmittels durchgeführt werden. Besonders geeignet sind Ether, Wasser und Gemische davon. Beispiele hierfür sind Dimethylformamid (DMF), Tetrahydrofuran (THF) und Diethylisophthalat (DEIP).

Formaldehyd kann dem erfindungsgemäßen Verfahren in Form von monomerem Formaldehyd und/oder in Form von höheren Homologen, sogenannten Poly(oxymethylen)glykolen, zugeführt werden. Das molare Verhältnis Anilin : Formaldehyd beträgt im allgemeinen 1,5 bis 10 : 1, bevorzugt 2 bis 5 : 1. Ferner ist die Umsetzung sowohl kontinuierlich als auch diskontinuierlich, in einem Batch-oder Semibatch-Verfahren möglich.

Im Schritt B) erfolgt die Phosgenierung des Roh-MDA Gemisches aus Schritt A) in an sich dem Fachmann bekannter Weise, beispielsweise wie in "Chemistry and Technology of Isocyanates" von H. Ulrich, John Wiley Verlag, 1996 und der darin zitierten Literatur beschrieben. Als Lösungsmittel können alle für den Phosgenierungsprozess bekannten inerten aromatischen oder aliphatischen Kohlenwasserstoffe bzw. Halogenkohlenwasserstoffe eingesetzt werden, in denen das jeweilige Isocyanat löslich ist und, die durch die Reaktionsbedingungen nicht angegriffen werden. Vorzugsweise werden aromatische Verbindungen wie z.B. Mono- oder o-Dichlorbenzol bzw. Toluol eingesetzt. Ebenfalls kann das Isocyanat, das hergestellt werden soll, selbst als Lösungsmittel dienen.

Anschließend an die Phosgenierung findet bevorzugt eine Aufarbeitung des Roh-Isocyanates statt, bei der überschüssiges Phosgen und Lösungsmittel abgetrennt werden. In einer möglichen Ausführungsform kann sich auch noch eine physikalische, z.B. thermische und/oder eine chemische Nachbehandlung zur Entfernung störender Nebenprodukte anschließen, wie sie z.B. in US 3912600, DD 288599, US 5364958, EP 0133538, JP 06345707, DD 288598, DD 288593, EP 0524507 und EP 0866057 beschrieben sind. Die Aufarbeitung und Nachbehandlung fällt im Rahmen dieser Anmeldung unter Schritt B.

Die Umsetzung in Schritt B) ergibt ein Gemisch aus verschiedenen MDI-Oligomeren und Isomeren. Dieses Gemisch ist allgemein bekannt unter dem Begriff "Roh-MDI". In Schritt C) und D) des erfindungsgemäßen Verfahrens erfolgt eine Auftrennung des Roh-MDIs. Dies kann über bekannte Verfahren wie beispielsweise Destillation, Lösungsmittelextraktion, Extraktion mit überkritischen Medien, wie z.B. überkritischem CO₂, oder Kristallisation erfolgen. Die Destillation ist bevorzugt.

In Schritt C) des erfindungsgemäßen Verfahrens wird mindestens ein Teil des Monomer-MDI aus dem in Schritt B) erhaltenen Roh-MDI abgetrennt, bevorzugt abdestilliert. Der bei dieser Abtrennung zurückbleibende Teil wird allgemein als Polymer-MDI (PMDI) bezeichnet. Die abgetrennte Menge von Monomer-MDI hängt von der Zusammensetzung ab, die das zurückbleibende PMDI aufweisen soll und liegt bei 10 bis 70 Gew.% und bevorzugt 20 bis 50 Gew.% bezogen auf die Menge an Roh-MDI. Die Destillatfraktion, bestehend aus Monomer-MDI wird anschließend in Schritt D), insbesondere zur Abtrennung von 2,4'-MDI, weiterverarbeitet. Das zurückbleibende PMDI kann ebenfalls zur Polyurethanherstellung verwendet werden. Da es sich bei Polyurethanen um aufeinander abgestimmte Systeme aus Isocyanat und Polyol handelt, ist es wünschenswert, dass das PMDI stets einen konstanten Gehalt an Monomer-MDI und PMDI aufweist, d.h. dass unterschiedliche Chargen keine unterschiedlichen Eigenschaften aufweisen.

Im Schritt D) wird das aus Schritt C) erhaltene Monomer-MDI, im wesentlichen enthaltend 2,4'-MDI und 4,4'-MDI durch mindestens teilweises Abtrennen von 2,4'-MDI aufgetrennt. Hierbei werden 10 bis 80 Gew.%, bevorzugt 15 bis 70, insbesondere 30 bis 70 Gew.% des 2,4'-MDIs, bezogen auf die Gesamtmenge an 2,4'-MDI, die in dem aus Schritt C) erhaltenen Monomer-MDI enthalten ist, abgetrennt. Nach der Abtrennung bleibt ein Gemisch zurück, dass 4,4'-MDI enthält oder es bleiben zwei oder mehr Gemische zurück, wobei das erste 4,4'-MDI enthält und die restlichen Gemische 4,4'-MDI und 2,4'-MDI in gegebenenfalls unterschiedlichen Anteilen enthalten.

In einer möglichen Ausführungsform erfolgt die Auftrennung bevorzugt in einer Destillationskolonne, wobei im Sumpf 4,4'-MDI und als Kopfprodukt 2,4'-MDI erhalten wird.

In einer bevorzugten Ausführungsform erfolgt die Auftrennung in einer Destillationskolonne, wobei im Sumpf 4,4'-MDI, als Kopfprodukt 2,4'-MDI und in einem Seitenabzug der Kolonne ein Gemisch aus 2,4'-/4,4'-MDI erhalten wird.

In einer besonders bevorzugten Ausführungsform enthält dieses Gemisch 30 bis 70 Gew.%, bevorzugt etwa 50 Gew.% 2,4'-MDI und 30 bis 70 Gew.%, bevorzugt etwa 50 Gew.% 4,4'-MDI. In dieser Ausführungsform erhält man üblicherweise, bezogen auf die Gesamtmenge an aus Schritt C erhaltenen Monomer-MDI,
1 bis 90 Gew.%, bevorzugt 40 bis 80 Gew.% 4,4'-MDI,
1 bis 80 Gew.%, bevorzugt 5 bis 30 Gew.% eines etwa 50/50 Gemisches aus 2,4'-MDI und 4,4'-MDI und
1 bis 50 Gew.%, bevorzugt 2 bis 20 Gew.% 2,4'-MDI.

In einer weiteren Ausführungsform kann in einer ersten Destillationskolonne das Gemisch aus C) in reines 4,4'-MDI und ein Gemisch aus 2,4'-/4,4'-MDI aufgetrennt werden. Dieses Gemisch aus 2,4'-MDI und 4,4'-MDI kann einer weiteren Destillation unterworfen werden, in der das 2,4'-MDI abgetrennt wird. Als Rückstand bleibt 4,4'-MDI zurück.

In einer weiteren Ausführungsform kann man die Trennung der Isomeren wie sie aus Schritt C) erhalten werden auch durch Kristallisation wie z.B. in DE 2322574, GB 1417087, US 4014914, BE 215525, DE 2606364, DE 19645659 beschrieben oder via Lösungsmittelextraktion wie z.B. DD 118618, US 4876380, US 4871460, DE 4200236 beschrieben, durchführen.

In einer bevorzugten Ausführungsform erfolgt die Auftrennung gemäß Schritt D) in einer Destillationskolonne. Ferner ist bevorzugt, dass Trennschritt C) durch Destillation und Trennschritt D) durch Kristallisation und/oder Lösungsmittelextraktion erfolgen. Ebenfalls ist es möglich, dass Trennschritt C) Kristallisation und/oder Lösungsmittelextraktion und Trennschritt D) durch Destillation erfolgt.

Gegenüber den Verfahren des Standes der Technik zeigt das erfindungsgemäße Verfahren folgende Vorteile: 2,4'-MDI wird als Isocyanat in größeren Mengen zur Verfügung gestellt, ohne dabei die Zusammensetzung des Produktmixes der Anlage, insbesondere die Zusammensetzung des gleichzeitig anfallenden Koppelproduktes PMDI wesentlich zu verändern und ohne dass eine grundlegende Änderung in den bestehenden Verfahren zur Herstellung von Isocyanaten durch Phosgenierung notwendig ist. Außerdem ermöglicht es das erfindungsgemäße Verfahren, die notwendige Menge an Säure (Katalysator) im MDA-Prozess zu verringern. Als Folge davon wird erstens weniger Natronlauge zur anschließenden Neutralisation der Säure benötigt, d.h. Einsparung von Kosten für die notwendige Lauge sind möglich und zweitens fällt eine geringere Salzfracht im Abwasser an, d.h. es erfolgt eine geringere Belastung für die Umwelt.

Für die Herstellung von Polyurethansystemen ist eine konstante Zusammensetzung von PMDI wichtig. Mit dem erfindungsgemäßen Verfahren lassen sich gezielt konstante Zusammensetzungen bezüglich der Oligomeren- und Isomerenverteilung des PMDI einstellen. So strebt man z.B. in Hartschaumanwendungen einen hohen Monomer-MDI Gehalt an, um ein gutes Fließverhalten zu erzielen. Darüberhinaus ist jedoch ein niedriger und konstanter Gehalt an 2,4'-MDI entscheidend, um ein gutes Durchhärtungsverhalten zu erreichen. Zusätzlich erhält man auf Grund des niedrigen 2,4'-MDI Gehaltes eine gute Vernetzung im Schaum und damit eine hohe Druckfestigkeit. Das in Schritt C) erhaltene PMDI weist daher bevorzugt einen 2,4'-MDI Gehalt von 1 bis 6 Gew.%, mehr bevorzugt 1,5 bis 4 Gew.-%, insbesondere 2 bis 4 Gew.% auf, damit lassen sich beispielsweise sensible Hartschaumsysteme vorteilhaft, reproduzierbar und zuverlässig herstellen.

Die Erfindung soll anhand der nachfolgenden Beispiele veranschaulicht werden.

### Vergleichsbeispiel:

In einem kontinuierlichen Prozess wird Anilin mit wässriger Formaldehydlösung im Verhältnis A/F = 2,2 zur Reaktion gebracht. Wässrige, konzentrierte Salzsäure wird dabei als Katalysator zugegeben. Das Verhältnis Säure zu Anilin beträgt 0,25. Das so erhaltene Reaktionsgemisch wird in an sich bekannter Weise aufgearbeitet, d.h. das Rohgemisch wird zuerst mit Natronlauge neutralisiert und anschließend mit Wasser salzfrei gewaschen. Danach wird zuerst Wasser und dann unumgesetztes Anilin abdestilliert. Das so erhaltene wasserfreie Roh-MDA (5,95 t/h) wird mit 8,5 t/h Phosgen in Chlorbenzol als Prozesslösungsmittel bei 120°C in Rührkesseln zu Isocyanat umgesetzt. Das die Phosgenierung verlassende Gemisch wird entsprechend dem Stand der Technik vom Phosgen und Chlorbenzol befreit und thermisch nachbehandelt. Es werden ca. 7,5 t/h Roh-MDI erhalten.
Anschließend wird von dem erhaltenen Roh-MDI 25 % (1.86 t/h) Monomer-MDI destillativ abgetrennt, so dass 5,64 t/h Polymer-MDI zurückbleibt. Von diesem Produkt wurden die Isocyanat-Kenndaten bestimmt und in Tabelle 1 angegeben. In Tabelle 2 ist die Oligomerenzusammensetzung angegeben. Das 2-Kern MDI wird in einer Kolonne einer weiteren Destillation unterworfen, dabei werden im Sumpf der Kolonne 1,6 t/h reines 4,4'-MDI erhalten. Zusätzlich erhält man 0,26 t/h eines 50/50-Gemisches aus 2,4'-MDI und 4,4'-MDI. Reines 2,4'-MDI wird in diesem Verfahren nicht gewonnen.

### Beispiel 1 gemäß dem erfindungsgemäßen Verfahren, S/A = 0,14:

In einem großtechnischen, kontinuierlichen Prozess wird Anilin mit wässriger Formaldehydlösung im Verhältnis A/F = 2,3 zur Reaktion gebracht. Wässrige, konzentrierte Salzsäure wird als Katalysator zugegeben. Das Verhältnis Säure zu Anilin beträgt 0,14. Das so erhaltene Reaktionsgemisch wird in an sich bekannter Weise aufgearbeitet, d.h. das Rohgemisch wird zuerst mit Natronlauge neutralisiert und anschließend mit Wasser salzfrei gewaschen. Danach wird zuerst Wasser und dann unumgesetztes Anilin abdestilliert. Das so erhaltene wasserfreie Roh-MDA (5,95 t/h) wird mit 8,5 t/h Phosgen in Chlorbenzol als Prozesslösungsmittel bei 120°C in Rührkesseln zum Isocyanat umgesetzt. Das die Phosgenierung verlassende Gemisch wird entsprechend dem Stand der Technik vom Phosgen und Chlorbenzol befreit und thermisch nachbehandelt. Es werden ca. 7,5 t/h Roh-MDI erhalten. Anschließend wird von dem erhaltenen Roh-MDI 25 % (1,86 t/h) 2-Kern MDI destillativ abgetrennt, so dass 5,64 t/h Polymer MDI zurückbleibt. Von diesem Produkt wurden die Isocyanat-Kenndaten bestimmt, diese Kenndaten finden sich in Tabelle 1. In Tabelle 2 ist die Oligomerenzusammensetzung angegeben. Das 2-Kern MDI wird in einer Kolonne einer weiteren Destillation unterworfen, dabei werden im Sumpf der Kolonne 1,42 t/h reines 4,4'-MDI erhalten. Zusätzlich erhält man 0,44 t/h eines 50/50-Gemisches aus 2,4'-MDI und 4,4'-MDI. 0,26 t/h dieses Gemisches werden direkt in einen Lagertank abgefüllt und verkauft. Die restlichen 0,18 t/h werden einer weiteren Destillation unterworfen, dabei werden ca. 0.09 t/h reines 2,4'-MDI produziert. Zusätzlich fallen ca. 0,09 t/h reines 4,4'-MDI an, die zu dem oben erhaltenen 4,4'-MDI Strom von 1,42 t/h zugemischt werden, so dass man insgesamt 1,51 t/h 4,4'-MDI erhält.

### Beispiel 2 gemäß dem erfindungsgemäßen Verfahren, S/A = 0,09:

In einem kontinuierlichen Prozess wird Anilin mit wässriger Formaldehydlösung im Verhältnis A/F = 2,3 zur Reaktion gebracht. Wässrige, konzentrierte Salzsäure wird als Katalysator zugegeben. Das Verhältnis Säure zu Anilin beträgt 0,09. Das so erhaltene Reaktionsgemisch wird in an sich bekannter Weise aufgearbeitet, d.h. das Rohgemisch wird zuerst mit Natronlauge neutralisiert und anschließend mit Wasser salzfrei gewaschen. Danach wird zuerst Wasser und dann unumgesetztes Anilin abdestilliert. Das so erhaltene wasserfreie Roh-MDA (1 kg/h) wird mit 1,4 kg/h Phosgen in Chlorbenzol als Prozesslösungsmittel bei 120°C in einem Rührkessel zum Isocyanat umgesetzt. Das die Phosgenierung verlassende Gemisch wird entsprechend dem Stand der Technik vom Phosgen und Chlorbenzol befreit und thermisch nachbehandelt. Es werden ca. 1,26 kg/h Roh-MDI erhalten.
Anschließend wird von dem erhaltenen Roh-MDI 25 % (316 g/h) 2-Kern MDI destillativ abgetrennt, so dass 944 g/h Polymer MDI zurückbleibt. Von diesem Produkt wurden die Isocyanat-Kenndaten bestimmt, diese Kenndaten finden sich in Tabelle 1. In Tabelle 2 ist die Oligomerenzusammensetzung angegeben. Das 2-Kern MDI wird in einer Kolonne einer weiteren Destillation unterworfen, dabei werden im Sumpf der Kolonne 204 g/h reines 4,4'-MDI erhalten. Zusätzlich erhält man 112 g/h eines 50/50-Gemisches aus 2,4'-MDI und 4,4'-MDI. 44 g/h dieses Gemisches werden direkt in einen Vorratsbehälter abgefüllt. Die restlichen 68 g/h werden einer weiteren Destillation unterworfen, dabei werden ca. 34 g/h reines 2,4'-MDI produziert. Die Isocyanat-Kenndaten des 2,4'-MDI sind in Tabelle 3 angegeben. Zusätzlich fallen ca. 34 g/h reines 4,4'-MDI an, die zu dem oben erhaltenen 4,4'-MDI Strom von 204 g/h zugemischt werden, so dass man insgesamt 238 g/h 4,4'-MDI erhält.

**Tabelle 1: Charakterisierung des PMDI**

| | NCO [%] | Viskosität [mPas] | 2,4"-MDI [%] | TC [ppm] | EHC [ppm] | DHC [ppm] |
|---|---|---|---|---|---|---|
| Vergleich | 31,65 | 180 | 2,15 | 1400 | 85 | 1050 |
| Beispiel 1 | 31,85 | 180 | 2,15 | 1350 | 85 | 1000 |
| Beispiel 2 | 31,85 | 180 | 2,2 | 1500 | 100 | 1100 |

**Tabelle 2: Oligomeren-Zusammensetzung des PMDI**

| | 2,4'-MDI [%] | 4,4'-MDI [%] | Σ 2-Kern MDI [%] | Σ 3-Kern MDI [%] | Σ 4-Kern MDI [%] |
|---|---|---|---|---|---|
| Vergleich | 2,15 | 36,7 | 38,88 | 29,03 | 8,50 |
| Beispiel 1 | 2,15 | 35,9 | 38,10 | 29,38 | 8,33 |
| Beispiel 2 | 2,2 | 35,6 | 37,8 | 29,68 | 8,4 |

**Tabelle 3: Kenndaten des 2,4'-MDI**

| | |
|---|---|
| TC [ppm] | 87 |
| NCO [%] | 33,5 |
| 2,4'-MDI Gehalt* [%] | 99,5 |
| 4,4'-MDI Gehalt* [%] | 0,23 |

| | |
|---|---|
| * Gaschromatographische Bestimmung | |

**Tabelle 4: Massenbilanz, Mengenströme**

| | Roh-MDI | PMDI | 4,4'-MDI | 2,4'-MDI | 50/50-Gemisch 2,4'-/4,4'-MDI |
|---|---|---|---|---|---|
| Vergleich | 7,5 t/h | 5,64 t/h | 1,6 t/h | 0 t/h | 0,26 t/h |
| | (100 %) | (75.2 %) | (21.3 %) | | (3.5 %) |
| Beispiel 1 | 7,5 t/h | 5,64 t/h | 1,51 t/h | 0,09 t/h | 0,26 t/h |
| | (100 %) | (75.2 %) | (20.1 %) | (1.2%) | (3.5 %) |
| Beispiel 2 | 1260 g/h | 944 g/h | 238 g/h | 34 g/h | 44 g/h |
| | (100 %) | (74.9 %) | (18.9 %) | (2.7 %) | (3.5 %) |

- EHC: Acidität (easily hydrolysable chlorine), gemessen entsprechend ASTM D 4667-87
- DHC: Hydrolysierbares Chlor (difficultly hydrolysable chlorine), gemessen nach ASTM D 4663-87.
- TC: Gesamtchlorgehalt, bestimmt mit Röntgenfluoreszenzanalyse
- NCO: Isocyanatgehalt, gemessen nach DIN 53185
- Viskosität: Bestimmung nach DIN 51550

## Patentansprüche

1. Verfahren zur Herstellung von MDI, umfassend folgende Schritte:
A) Umsetzung von Anilin und Formaldehyd in Gegenwart einer Säure zu Methylen(diphenyldiaminen) und Polymethylen-polyphenylen-polyaminen, wobei das molare Verhältnis von Säure zu Amin maximal 0,2 ist,
B) Umsetzung des in Schritt A) erhaltenen Gemisches mit Phosgen zu Methylen(diphenyldiisoeyanaten) und Polymethylen-polyphenylen-polyisocyanaten,
C) Auftrennen des aus Schritt B) erhaltenen Gemisches in Monomer-MDI und Polymer-MDI, wobei die abgetrennte Menge von Monomer-MDI 10 bis 70 Gew.-%, bezogen auf die Menge an Roh-MDI beträgt, und
D) Auftrennen des in Schritt C) erhaltenen Monomer-MDI unter Abtrennen von 2,4'-MDI, wobei 10 bis 80 Gew.-% 2,4'-MDI, bezogen auf die Gesamtmenge an 2,4'-MDI, die in dem aus Schritt C erhaltenen Monomer-MDI enthalten ist, abgetrennt wird.

2. Verfahren nach Anspruch 1, **dadurch gekennzeichnet, dass** die Trennschritte C) und D) durch Destillation erfolgen.

3. Verfahren nach Anspruch 1, **dadurch gekennzeichnet, dass** die Trennschritte C) und D) durch-Kristallisation und/oder Lösungsmittelextraktion erfolgen.

4. Verfahren nach Anspruch 1, **dadurch gekennzeichnet, dass** Trennschritt C) durch Destillation und Trennschritt D) durch Kristallisation und/oder Lösungsmittelextraktion erfolgen.

5. Verfahren nach einem der Ansprüche 1 bis 4, **dadurch gekennzeichnet, dass** das in Schritt C erhaltene Polymer-MDI einen Gehalt an 2,4'-MDI von 1 bis 6 Gew.% aufweist.

6. Verfahren nach einem der Ansprüche 1 bis 5, **dadurch gekennzeichnet, dass** in Schritt D) das aus Schritt C) erhaltene Monomer-MDI in die Bestandteile
D1) 4,4'-MDI und
D3) 2,4'-MDI aufgetrennt wird.

7. Verfahren nach einem der Ansprüche 1 bis 5, **dadurch gekennzeichnet, dass** in Schritt D) das aus Schritt C) erhaltene Monomer-MDI in die Bestandteile
D1) 4,4'-MDI,
D2) Gemisch aus 2,4'-MDI und 4,4'-MDI und
D3) 2,4'-MDI aufgetrennt wird.

8. Verfahren nach Anspruch 7, **dadurch gekennzeichnet, dass** das Gemisch D2) zu jeweils etwa 50 Gew.-% aus 2,4'-MDI und 4,4'-MDI besteht.

## Claims

1. A process for the preparation of MDI, comprising the following steps:
A) reaction of aniline and formaldehyde in the presence of an acid to give methylene(diphenylediamines) and polymethylenepolyphenylenepolyamines, the molar ratio of acid to amine being not more than 0.2,
B) reaction of the mixture obtained in step A) with phosgene to give methylene(diphenyl diisocyanates) and polymethylenepolyphenylene polyisocyanates,
C) separation of the mixture obained in step B) into monomeric MDI and polymeric MDI, the amount of monomeric MDI separated off being from 10 to 70% by weight, based on the amount of crude MDI, and
D) separation of the monomeric MDI obtained in step C) with isolation of 2,4'-MDI, from 10 to 80% by weight of 2,4'-MDI, based on the total amount of 2,4'-MDI present in the monomeric MDI obtained from step C, being separated off.

2. The process according to claim 1, wherein the separation steps C) and D) are carried out by distillation.

3. The process according to claim 1, wherein the separation steps C) and D) are carried out by crystallization and/or solvent extraction.

4. The process according to claim 1, wherein separation step C) is carried out by distillation and separation step D) by crystallization and/or solvent extraction.

5. The process according to any of claims 1 to 4, wherein the polymeric MDI obtained in step C has a 2,4'-MDI content of from 1 to 6% by weight.

6. The process according to any of claims 1 to 5, wherein, in step D), the monomeric MDI obtained in step C) is separated into the components
D1) 4,4'-MDI and
D3) 2,4'-MDI.

7. The process according to any of claims 1 to 5, wherein, in step D), the monomeric MDI obtained in step C) is separated into the components
D1) 4,4'-MDI,
D2) a mixture of 2,4'-MDI and 4,4'-MDI and
D3) 2,4'-MDI.

8. The process according to claim 7, wherein the mixture D2) consists of about 50% by weight each of 2,4'-MDI and 4,4'-MDI.

## Revendications

1. Procédé de préparation de MDI, comprenant les étapes suivantes :
A) une réaction d'aniline et de formaldéhyde en présence d'un acide pour former des méthylène-(diphényldiamines) et des polyméthylène-polyphénylène-polyamines, le rapport molaire entre acide et amine étant au maximum de 0,2,
B) une réaction du mélange obtenu dans l'étape A) avec du phosgène pour former des méthylène-(diphényldiisocyanates) et des polyméthylène-polyphénylène-polyisocyanates,
C) une séparation du mélange obtenu dans l'étape B) en MDI monomère et MDI polymère, la quantité séparée de MDI monomère étant de 10 à 70 % en poids, par rapport à la quantité de MDI brut, et
D) une séparation du MDI monomère obtenu dans l'étape C) avec isolement de 2,4'-MDI, 10 à 80 % en poids de 2,4'-MDI, par rapport à la quantité totale de 2,4'-MDI contenu dans le MDI monomère obtenu dans l'étape C), étant isolés.

2. Procédé suivant la revendication 1, **caractérisé en ce que** les étapes de séparation C) et D) ont lieu par distillation.

3. Procédé suivant la revendication 1, **caractérisé en ce que** les étapes de séparation C) et D) ont lieu par cristallisation et/ou par extraction par solvant.

4. Procédé suivant la revendication 1, **caractérisé en ce que** l'étape de séparation C) a lieu par distillation et l'étape de séparation D) par cristallisation et/ou extraction par solvant.

5. Procédé suivant l'une des revendications 1 à 4, **caractérisé en ce que** le MDI polymère obtenu dans l'étape C) présente une teneur en 2,4'-MDI de 1 à 6 % en poids.

6. Procédé suivant l'une des revendications 1 à 5, **caractérisé en ce que**, dans l'étape D), le MDI monomère obtenu dans l'étape C) est séparé en les composants
D1) 4,4'-MDI, et
D3) 2,4'-MDI.

7. Procédé suivant l'une des revendications 1 à 5, **caractérisé en ce que**, dans l'étape D), le MDI monomère obtenu dans l'étape C) est séparé en les composants
D1) 4,4'-MDI,
D2) un mélange de 2,4'-MDI et de 4,4'-MDI, et
D3) 2,4'-MDI.

8. Procédé suivant la revendication 7, **caractérisé en ce que** le mélange D2) est constitué respectivement d'environ 50 % en poids de 2,4'-MDI et de 4,4'-MDI.
